(19) Europäisches Patentamt
European Patent Office
Office européen des brevets

(11) Publication number: **0 179 131**
**B1**

(12) **EUROPEAN PATENT SPECIFICATION**

(45) Date of publication of patent specification: **10.01.90**

(51) Int. Cl.$^5$: **C 07 C 2/00**

(21) Application number: **85902267.5**

(22) Date of filing: **11.04.85**

(86) International application number:
**PCT/US85/00650**

(87) International publication number:
**WO 85/04866 07.11.85 Gazette 85/24**

(54) **METHANE CONVERSION.**

(30) Priority: **16.04.84 US 600665**
**16.04.84 US 600669**
**16.04.84 US 600730**
**16.04.84 US 600917**
**16.04.84 US 600918**
**31.10.84 US 666694**
**31.10.84 US 666700**
**05.11.84 US 668230**
**05.11.84 US 668299**

(43) Date of publication of application:
**30.04.86 Bulletin 86/18**

(45) Publication of the grant of the patent:
**10.01.90 Bulletin 90/02**

(84) Designated Contracting States:
**BE DE FR NL**

(56) References cited:
**SU-A- 747 847**
**US-A-1 206 156**
**US-A-4 205 194**
**US-A-4 239 658**
**US-A-4 443 645**
**US-A-4 443 648**
**US-A-37 905 01**

(73) Proprietor: **ATLANTIC RICHFIELD COMPANY**
**515 South Flower Street**
**Los Angeles California 90071 (US)**

(72) Inventor: **GAFFNEY, Anne, M.**
**611 Owen Road**
**West Chester, PA 19380 (US)**
Inventor: **WITHERS, Howard, P.**
**316 Laurelwood Drive**
**Douglassville, PA 19518 (US)**

(74) Representative: **Cropp, John Anthony David et al**
**MATHYS & SQUIRE 10 Fleet Street**
**London, EC4Y 1AY (GB)**

(56) References cited:
**N, Chem. Abstracts 93:87461t**

**N, Journal of Catalysis; issued 1982 G.E. Keller and M.M. Bhasin, Synthesis of Methane via Oxidative coupling of Methane, see pages 9-18**

**N, Journal of the Chinese Chemical Society, Trelant Fang Chuin-Tih Yeh, Catalytic Pyrclysisct Methane see pages 265-273**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 179 131 B1**

**Description**

Background of the Invention

Field of the Invention

This invention relates to systhesis of hydrocarbons from a methane source. A particular application of this invention is a method for converting natural gas to a more readily transportable material.

Description of the Prior Art

A major source of methane is natural gas. Other sources of methane have been considered for fuel supply, e.g., the methane present in coal deposits or formed during mining operations. Relatively small amounts of methane are also produced in various petroleum processes.

The composition of natural gas at the wellhead varies but the major hydrocarbon present is methane. For example, the methane content of natural gas may vary within the range from about 40 to about 95 volume percent. Other constituents of natural gas include ethane, propane, butanes, pentane (and heavier hydrocarbons), hydrogen sulfide, carbon dioxide, helium and nitrogen.

Natural gas is classified as dry or wet depending upon the amount of condensable hydrocarbons contained in it. Condensable hydrocarbons generally comprise $C_3$+ hydrocarbons although some ethane may be included. Gas conditioning is required to alter the composition of wellhead gas, processing facilities usually being located in or near the production fields. Conventional processing of wellhead natural gas yields processed natural gas containing at least a major amount of methane.

Large-scale use of natural gas often requires a sophisticated and extensive pipeline system. Liquefaction has also been employed as a transportation means, but processes for liquefying, transporting, and revaporizing natural gas are complex, energy-intensive and require extensive safety precautions. Transport of natural gas has been a continuing problem in the exploitation of natural gas resources. It would be extremely valuable to be able to convert methane (e.g. natural gas) to more readily handleable or transportable products. Moreover, direct conversion to olefins such as ethylene or propylene would be extremely valuable to the chemical industry.

Recently it has been discovered that methane may be converted to higher hydrocarbons by a process which comprises contacting methane and an oxidative synthesizing agent at synthesizing conditions (e.g. at a temperature selected within the range from about 500° to about 1000°C). Oxidative synthesizing agents are compositions having as a principal component at least one oxide of at least one metal which compositions produce $C_2$+ hydrocarbon products, co-product water, and a composition comprising a reduced metal oxide when contacted with methane at synthesizing conditions. Reducible oxides of several metals have been identified which are capable of converting methane to higher hydrocarbons. In particular, oxides of manganese, tin, indium, germanium, lead, antimony and bismuth are most useful. See U.S. Patent Numbers 4,443,649; 4,444,984; 4,443,648; 4,443,645; 4,443,647; 4,443,644; and 4,443,646.

US—A—1206156, published in 1916 describes a process in which the thermal units in and illuminating efficiency of natural gas are improved by contact with a catalyst formed by calcining a mixture of a metal of the iron group e.g. Fe, an alkaline earth and an alkali. In the exemplified catalyst composition, the atomic ratio of Fe to alkali metal plus alkaline earth metal is low, at 0.2 : 1 or less, and the major product of the conversion is acetylene.

SU—A—747847 describes a process for converting methane into liquid hydrocarbons, mainly $C_{10}$—$C_{12}$ paraffins, by contacting the methane at a temperature in the range 200 to 400°C with a NaY zeolite dosed with a mixture of lanthanides of unspecified composition.

The pyrolysis of methane in the presence of catalytic materials comprising certain metal oxides, including those of Fe and Ce, is reported in the Journal of the Chinese Chemical Society (1981) pages 265 to 273. The catalytic materials do not contain alkali or alkaline earth metals in conjunction with these metal oxides.

It has now been found that methane may be converted to higher hydrocarbon products by contacting a methane-containing gas at elevated temperature with a contact solid comprising at least one reducible oxide of at least one metal selected from praseodymium, terbium, cerium, iron and ruthenium.

In accordance with one aspect of the invention, the contact solid comprises at least one reducible oxide (as hereinafter defined) of at least one metal selected from Pr, Tb and Ru and the temperature of the contacting is in the range of 700 to 900°C.

In accordance with another aspect of the invention, the contact solid comprises (i) at least one reducible oxide (as hereinafter defined) of at least one metal selected from Pr, Tb, Ru, Fe and Ce, and (ii) at least one promoter selected from alkali metals, alkaline earth metals, and compounds thereof and the contacting is effected at a temperature in the range of 500 to 1000°C. Where the reducible oxide is provided solely by an oxide of Fe, the atomic ratio of Fe to alkali metal and/or alkaline earth metal provided by the promoter must be at least one.

In accordance with one embodiment of the invention, a method for synthesizing hydrocarbon from a methane source comprises the steps of

(a) contacting a gas comprising methane with the contact solid at the contacting temperature to produce $C_2$+ hydrocarbons, coproduct water and solids comprising reduced metal oxide;

(b) recovering $C_2$+ hydrocarbons;

(c) at least periodically contacting solids comprising reduced metal oxide with an oxygen-containing gas to produce solid comprising reducible metal oxide; and

(d) contacting a gas comprising methane with solids produced in step (c) as recited in step (a).

In the application of this embodiment to the first aspect of the invention, the contacting of step (a) is effected in the substantial absence of catalytically effective Ni, Rh, Pd, Ag, Os, Ir, Pt, Au and compounds thereof.

In the application of the embodiment to the second aspect of the invention, there is no limit on the atomic ratio of Fe to alkali metal and/or alkaline earth metal provided by the promoter.

Alkali metals are selected from the group consisting of Li, Na, K, Rb and Cs. Alkaline earth metals are selected from the group consisting of Mg, Ca, Sr and Ba.

The atomic ratio of the reducible metal oxide (expressed as the metal, e.g., Pr) to alkali or alkaline earth metal (where used) is desirably within the range of about 1—15:1. Hydrocarbons produced by the process may include lower alkanes, lower olefins and aromatics. The reducible metal oxide is reduced by contact with methane and is reoxidizable by contact with an oxygen-containing gas.

Incorporating an alkali or alkaline earth metal into the contact solid substantially reduces the formation of combustion products and improves higher hydrocarbon product selectivity.

Detailed Description of the Invention

Reducible oxides of Pr, Tb, Ce, Fe and Ru can be supplied from a variety of known sources. The term "reducible" is used to identify those oxides which are reduced by contact with methane at the contacting temperature. Preferred oxides include $Pr_6O_{11}$, $Tb_4O_7$, $CeO_2$, $Fe_2O_3$, $Fe_3O_4$, and ruthenium dioxide. Of iron oxides, the bulk iron oxide $Fe_3O_4$ is particularly preferred.

The contact solid employed in the present process may also contain, in one embodiment of the present invention, at least one alkali metal or alkaline earth metal. Alkali metals are preferred. Sodium and lithium are presently preferred alkali metals. The amount of alkali/alkaline earth metal incorporated into the contact solid is not narrowly critical. The preferred atomic ratio of the reducible metal oxide component (expressed as the metal, e.g, Pr) to the alkali/alkaline earth metal component (expressed at the metal, e.g., Na) in the case of Pr, Tb and Ce is within the range of 1—15:1, more preferably within the range of 1—3:1; and in the case of Fe and Ru is within the range of 1—500:1, more preferably in the range of 2—100:1, still more preferably 2—10:1.

The term "reducible", as employed herein, is used to identify those oxides of the specified metals which are reduced by contacting methane at synthesizing conditions. The term "oxide(s) of metal(s)" means: (1) one or more metal oxides (i.e., compounds described by the general formula $M_xO_y$ wherin M is a metal and the subscripts x and y designate the relative atomic proportions of metal and oxygen in the composition) and/or (2) one or more oxygen-containing metal compounds, provided that such oxides and compounds have the capability of performing to product higher hydrocarbon products as set forth herein.

Oxidative synthesizing agents have previously been found to comprise reducible oxides of metals selected from the group consisting of Mn, Sn, In, Ge, Sb, Pb, and Bi and mixtures thereof. Particularly effective oxidative synthesizing agents have been found to comprise a reducible oxide of manganese and mixtures of a reducible oxide of manganese with other oxidative synthesizing agents. It is within the scope of the present invention to include other effective oxidative synthesizing agent components with the reducible metal oxides of the present invention. Thus, reducible oxides of metals selected from Pr, Tb, Ce, Fe and Ru may be combined with reducible oxide(s) of metal(s) selected from Mn, Sn, In, Ge, Sb, Pb, Bi and mixtures thereof.

It is also within the scope of the present invention to include at least one phosphorus component in the solid contacted with methane.

While the exact composition of the contact solids is more complex, a preferred group of solids employed in the process of this invention may be described by the following empirical expression:

$$A_a\, B_b\, C_c\, P_d\, O_e$$

wherein A is selected from the group consisting of Pr, Tb, Ce, Fe, Ru and mixtures thereof; B is selected from the group consisting of alkali and alkaline earth metals; C is selected from the group consisting of Mn, Sn, In, Ge, Pb, Sb, Bi and mixtures thereof; a, b, c, d and e indicate the atomic ratio of each component; and when a is 10, b is within the range of 0.5—10, c is within the range of 0—10, d is within the range of 0—10, and e has a value which is determined by the valences and proportions of other elements present.

These components may be associated with other support materials. However, in a presently preferred embodiment, a reducible oxide of metals selected from Pr, Tb, Ce, Fe and Ru is employed as a support for the other components of the solids. While use of other supports is within the scope of this invention, it has been found that materials such as silica and alumina tend to deactivate the solids of this invention via the formation of silicates and aluminates.

One particularly preferred embodiment of the present invention comprises contacting methane at a temperature within the range of 500 to 1000°C with a solid comprising a member of the group consisting of alkali metals and compounds thereof associated with a support comprising a reducible oxide of Pr, Tb, Fe or Ru. Preferably, the reducible oxide is selected from $Pr_6O_{11}$, $Tb_4O_7$, $Fe_3O_4$ and ruthenium dioxide. Still

more particularly, the presently preferred alkali metal associated with these supports is Na or Li, where the metal of the reducible oxide is Pr or Tb, or Na where the metal of the reducible metal oxide is Fe or Ru.

The contact solids employed in this invention can be prepared by any suitable method. Conventional methods such as precipitation, co-precipitation, impregnation, or dry-mixing can be used. Supported solids may be prepared by methods such as absorption, impregnation, precipitation, co-precipitation, and dry-mixing. When phosphorus is incorporated in the agent, it is desirable to provide it in the form of a phosphate of an alkali metal or an alkaline earth metal. Substantially any compound of these elements can be employed in the preparation of the contact solid.

A suitable method of preparation is to impregnate a support with solutions of compounds of the desired metals. Suitable compounds useful for impregnation include the acetates, acetylacetonates, oxides, carbides, carbonates, hydroxides, formates, oxalates, nitrates, phosphates, sulfates, sulfides, tartrates, fluorides, chlorides, bromides, or iodides. After impregnation the preparation is dried to remove solvent and the dried solid is prepared for use by calcining, preferably in air at a temperature selected within the range of 300 to 1200°C. Particular calcination temperatures will vary depending upon the particular metal compound of compounds employed.

If phosphorus is used, the alkali/alkaline earth metal (where used) and phosphorus are preferably added to the composition as compounds containing both P and alkaki/alkaline earth metals. Examples are the orthophosphates, metaphosphates, and pyrophosphates or alkali/alkaline earth metals. Pyrophosphates have been found to give desirable results. Sodium pyrophosphate is particularly preferred.

Regardless of how the components of the contact solid are combined, the resulting composite generally will be dried and calcined at elevated temperatures prior to use in the process of this invention.

The present process is distinguished from previously suggested methane conversion processes which rely primarily on interactions between methane and at least one of nickel and the noble metals, such as rhodium, palladium, silver, osmium, iridium, platinum and gold. An example of this type of process is disclosed in U.S. Patent 4,205,194. The present process does not require that methane be contacted with one or more of nickel and such noble metals and compounds thereof.

Moreover, in a preferred embodiment, such contacting is carried out in the substantial absence of catalytically effective nickel and the noble metals, such as Rh, Pd, Ag, Os, Ir, Pt and Au and compounds thereof to minimise the deleterious catalytic effects of such metals and compounds thereof. For example, at the conditions, e.g., temperatures, useful for the contacting step of the present invention, these metals when contacted with methane tend to promote coke formation, and the metal oxides when contacted with methane tend to promote formation of combustion products ($CO_x$) rather than the desired hydrocarbons. The term "catalytically effective" is used herein to identify that quantity of one or more of nickel and the noble metals and compounds thereof which when present substantially changes the distribution of products obtained in the contacting step of this invention relative to such contacting in the absence of such metals and compounds thereof.

In addition to methane, the feedstock employed in the method of this invention may contain other hydrocarbon or non-hydrocarbon components, although the methane content should typically be within the range of 40 to 100 volume percent, preferably from 80 to 100 volume percent, more preferably from 90—100 volume percent.

If reducible oxides of In, Ge or Bi are present in the solid, the particular operating temperature for the contacting of the methane-containing gas and the contact solid preferably does not exceed about 850°C, so as to minimise sublimation or volatilisation of the metal (or compound thereof).

Operating pressure for the methane contacting step is not critical to the presently claimed invention. However, both general system pressure and partial pressure of methane have been found to affect overall results. Preferred operating pressures are within the range of 1 to 100 bar (atmospheres), more preferably within the range of 1 to 30 bar (atmospheres).

Contacting methane and a reducible metal oxide to form higher hydrocarbons from methane also produced metal oxide and co-product water. The exact nature of the reduced metal oxides are unknown, and so they are referred to herein as "reduced metal oxides". Regeneration of a reducible metal oxide is readily accomplished by contacting such reduced materials with oxygen (e.g., an oxygen-containing gas such as air) at elevated temperatures, preferably at a temperature selected within the range of 300 to 1200°C, the particular temperature selected depending on the metal(s) included in the solid.

In carrying out the present process, a single reactor apparatus containing a fixed bed of solids may be used with intermittent or pulsed flow of a first gas comprising methane and a second gas comprising oxygen (e.g., oxygen, oxygen diluted with an inert gas, or air, preferably air). The methane contacting step and the oxygen contacting step may also be performed in physically separate zones with solids recirculating between the two zones.

Thus, a suitable method for synthesizing hydrocarbons from a methane source comprises: (a) contacting a gas comprising methane and particles comprising a reducible metal oxide to form higher hydrocarbon products, coproduct water, and reduced metal oxide; (b) removing particles comprising reduced metal oxide from the first zone and contacting the reduced particles in a second zone with an oxygen-containing gas to form particles comprising a reducible metal oxide and (c) returning the particles produced in the second zone to the first zone. The steps are preferably repeated at least periodically, and

more preferably the steps are continuous. In the more preferred embodiment solids are continuously circulated between at least one methane-contact zone and at least one oxygen-contact zone.

Particles comprising reducible metal oxide which are contacted with methane may be maintained as fluidized, ebullating, or entrained beds of solids. Preferably methane is contacted with a fluidized bed of solids.

Similarly, particles comprising reduced metal oxide which are contacted with oxygen may be maintained as fluidized, ebullating or entrained beds of solids. Preferably oxygen is contacted with a fluidized bed of solids.

In one more preferred embodiment of the present invention, methane feedstock and particles comprising contact solid are continuously introduced into a methane contact zone maintained at synthesizing conditions. Synthesizing conditions include the temperature and pressures described above. Gaseous reaction products from the methane contact zone (separated from entrained solids) are further processed, e.g., they are passed through a fractionating system wherein the desired hydrocarbon products are separated from unconverted methane and combustion products. Unconverted methane may be recovered and recycled to the methane contact zone.

Particles comprising reduced metal oxide are contacted with oxygen in an oxygen contact zone for a time sufficient to oxidize at least a portion of the reduced oxide to produce a reducible metal oxide and to remove, i.e., combust, at least a portion of any carbonaceous deposit which may form on the particles in the methane contact zone. The conditions of the oxygen contact zone will preferably include a temperature selected within the range of 300 to 1200°C, pressures of up to about 30 bar (atmospheres), and average particle contact time within the range of 1 to 120 minutes. Sufficient oxygen is preferably provided to oxidize all reduced metal oxide to produce a reducible oxide and to completely combust any carbonaceous deposit material deposited on the particles. At least a portion of the particles comprising reducible metal oxide which are produced in the oxygen contact zone are returned to the methane contact zone.

The rate of solids withdrawal from the methane contact zone is desirably balanced with the rate of solids passing from the oxygen contact zone to the methane contact zone so as to maintain a substantially constant inventory of particles in the methane contact zone, thereby enabling steady state operation of the synthesizing system.

In one alternative process employing the method of this invention, a gas comprising oxygen may be co-fed with a hydrocarbon gas comprising methane to the methane contact zone; see EPA 85902265.9 (EP—A—0179856).

In a further alternative process employing the method of this invention, the olefin content of the effluent produced by methane conversion as described herein may be oligomerized to product normally liquid higher hydrocarbon products; see EPA 85902268.3 (EP—A—0182811).

In a still further alternative process employing the method of this invention, it has been found advantageous to recover $C_2$+ alkanes from (1) the effluent produced by methane conversion as described herein and/or (2) streams derived from such effluent and to recycle such alkanes to the methane contact zone.

In a still further alternative process employing the method of this invention, it has been found that halogen and chalcogen promoters enhance results obtained when methane is converted to higher hydrocarbons by contact with a reducible metal oxide; see EPA 85902266.7 (EP—A—179857). $NO_x$ may also be used as promoters.

The invention is further illustrated by reference to the following examples

Methane-contact runs were made at about atmospheric pressure in quartz tube reactors (18 mm. inside diameter) packed with 10 ml. of contact solid. The reactors were brought up to temperature under a flow of nitrogen which was switched to methane at the start of the run. Unless otherwise indicated, all methane-contact runs described in the following examples has a duration of 2 minutes. At the end of each methane-contact run, the reactor was flushed with nitrogen and the solids were regenerated under a flow of air (usually at 800°C for 30 minutes). The reactor was then again flushed with nitrogen and the cycle repeated. Most of the results reported below are based on the cumulative sample collected after the contact solid has been through at least one cycle of methane contact and regeneration.

Experimental results reported below include conversions and selectivities calculated on a carbon mole basis. Carbonate selectivities were calculated from the $CO_2$ content of the $N_2$ purge gas collected after the methane contact run.

Space velocities are reported as gas hourly space velocities ($hr.^{-1}$) and are identified as "GHSV" in the Examples.

## Example 1

A contact solid comprising sodium/Pr oxide was prepared by impregnating $Pr_6O_{11}$ with the appropriate amount of sodium (as sodium acetate) from water solutions. The impregnated solids were dried at 110°C for 2 hours and then calcined in air by raising the temperature from 200°C to 800°C at a rate of about 100°/hour and then holding the temperature at 800°C for 16 hours. The calcined solids contained 4 wt.% Na. Results reported below in Table I are based on analyses of cumulative samples collected during a two-minute methane-contact run.

TABLE I

| | |
|---|---|
| Temp. (°C) | 825 |
| GHSV ($hr.^{-1}$) | 2400 |
| % $CH_4$ Conv. | 36.4 |
| % $C_2$ + Sel. | 51.2 |
| % $CO_x$ Sel. | 45.0 |
| % Coke Sel. | 1.1 |
| % Carbonate Sel. | 2.7 |

Example 2

The procedure described in Example 1 was repeated except that calcined $Pr_6O_{11}$ was contacted with methane in the absence of sodium. Results are shown in Table II below.

TABLE II

| | |
|---|---|
| Temp. (°C) | 825 |
| GHSV ($hr.^{-1}$) | 2400 |
| % $CH_4$ Conv. | 39.5 |
| % $C_2$ + Sel. | 1.0 |
| % $CO_x$ Sel. | 95.7 |
| % Coke Sel. | 1.5 |
| % Carbonate Sel. | 1.7 |

Example 3

A number of methane contact runs over 4 wt. % $Na/Pr_6O_{11}$ (prepared as described in Example 1) were performed to demonstrate the effect of temperatures and space velocities on process results. Results are shown in Table III below.

TABLE III

| | | | | | |
|---|---|---|---|---|---|
| Temp. (°C) | 750 | 750 | 750 | 825 | 825 |
| GHSV ($hr.^{-1}$) | 1200 | 2400 | 4800 | 4800 | 4800 |
| % $CH_4$ Conv. | 28.0 | 20.5 | 12.7 | 27.6 | 23.3 |
| % $C_2$ + Sel. | 64.3 | 76.0 | 84.1 | 62.6 | 69.4 |
| % $CO_x$ Sel. | 28.3 | 20.2 | 12.1 | 34.3 | 27.2 |
| % Coke Sel. | 1.2 | 0.8 | 1.0 | 0.9 | 1.2 |
| % Carbonate Sel. | 3.3 | 3.0 | 2.8 | 2.2 | 2.2 |

Example 4

A contact solid comprising $NaMnO_2$/Pr oxide was prepared by impregnating $Pr_6O_{11}$ with the appropriate amount of sodium permanganate from a water solution. The calcined solids contained the equivalent of 10 wt. % $NaMnO_2$. Results reported below in Table IV are based on analyses of cumulative samples collected during two-minute methane runs at a temperature of 825°C and 600 $hr.^{-1}$ GHSV.

TABLE IV

| | |
|---|---|
| % $CH_4$ Conv. | 80.9 |
| % $C_2$ + Sel. | 6.8 |
| % $CO_x$ Sel. | 89.9 |
| % Coke Sel. | 3.3 |

Example 5

A lithium/praseodymium oxide solid was prepared by impregnating $Pr_6O_{11}$ with the appropriate amount of lithium (as lithium acetate) from water solutions. The dried, calcined solid contained 1.2 wt.% Li. When the solid was contacted with methane at 775°C and 2400 hr.$^{-1}$ GHSV, the following results were obtained: 21.7% methane conversion and 74.3% $C_2$+ hydrocarbon selectivity. After 15 cycles of methane contact/air regeneration, the following results were obtained during the sixteenth methane contact run: 8.2% conversion and 89.4% $C_2$+ hydrocarbon selectivity.

Example 6

A potassium/praseodymium oxide solid was prepared by impregnating $Pr_6O_{11}$ with the appropriate amount of potassium (as potassium acetate) from water solutions. The dried, calcined solid contained 6.6 wt.% K. When the solid was contacted with methane at 775°C and 2400 hr.$^{-1}$ GHSV, the following results were obtained: 28.6% methane conversion and 50.9% $C_2$+ hydrocarbon selectivity.

Example 7

A contact solid comprising sodium/Tb oxide was prepared by impregnating $Tb_4O_7$ with the appropriate amount of sodium (as sodium acetate) from water solutions. The impregnated solids were dried at 110°C for 2 hours and then calcined in air by raising the temperature from 200°C to 800°C at a rate of about 100°C/hour and then holding the temperature at 800°C for 16 hours. The calcined solids contained 4 wt.% Na. Results reported below in Table V are based on analyses of cumulative samples collected during a two-minute methane-contact run.

TABLE V

| | |
|---|---|
| Temp. (°C) | 825 |
| GHSV (hr.$^{-1}$) | 1200 |
| % $CH_4$ Conv. | 30.0 |
| % $C_2$ + Sel. | 43.0 |
| % $CO_x$ Sel. | 53.6 |
| % Coke Sel. | 0.7 |
| % Carbonate Sel. | 2.8 |

Example 8

The procedure described in Example 7 was repeated except that calcined $Tb_4O_7$ was contacted with methane in the absence of sodium. Results are shown in Table IV below:

TABLE VI

| | |
|---|---|
| Temp. (°C) | 825 |
| GHSV (hr.$^{-1}$) | 1200 |
| % $CH_4$ Conv. | 20.0 |
| % $C_2$ + Sel. | 1.8 |
| % $CO_x$ Sel. | 94.9 |
| % Coke Sel. | 3.2 |
| % Carbonate Sel. | 0.1 |

Example 9

A number of methane contact runs over 4 wt.% $Na/Tb_4O_7$ (prepared as described in Example 7) were performed to demonstrate the effect of temperatures and space velocities on process results. Results are shown in Table VII below.

TABLE VI

| | | | | | | |
|---|---|---|---|---|---|---|
| Temp. (°C) | 750 | 750 | 825 | 825 | 850 | 875 |
| GHSV ($hr.^{-1}$) | 1200 | 3600 | 600 | 2400 | 600 | 600 |
| % $CH_4$ Conv. | 23.7 | 8.5 | 44.9 | 14.3 | 48.0 | 53.3 |
| % $C_2$ + Sel. | 56.7 | 71.5 | 30.3 | 46.3 | 18.8 | 21.8 |
| % $CO_x$ Sel. | 38.9 | 22.7 | 64.5 | 50.3 | 80.3 | 80.8 |
| % Coke Sel. | 0.8 | 0.8 | 1.1 | 0.7 | 0.4 | 2.3 |
| % Carbonate Sel. | 3.7 | 5.0 | 4.2 | 2.8 | 0.5 | 6.1 |

Example 10

A contact solid comprising soldium/Ce oxide was prepared by impregnating $CeO_2$ with the appropriate amount of sodium (as sodium acetate) from water solutions. The impregnated solids were dried at 110°C for 2 hours and then calcined in air by raising the temperature from 200°C to 800°C at a rate of about 100°C/hour and then holding the temperature at 800°C for 16 hours. The calcined solids contained 4 wt.%Na. Results reported below in Table VIII are based on analyses of cumulative samples collected during a two-minute methane-contact run.

TABLE VIII

| | |
|---|---|
| Temp. (°C) | 825 |
| GHSV ($hr.^{-1}$) | 1200 |
| % $CH_4$ Conv. | 4.6 |
| % $C_2$ + Sel. | 28.3 |
| % $CO_x$ Sel. | 32.4 |
| % Coke Sel. | 14.6 |
| % Carbonate Sel. | 24.8 |

Example 11 (Comparative)

The procedure described in example 10 was repeated except that calcined $CeO_2$ was contacted with methane in the absence of sodium. Results are shown in Table IX below.

TABLE IX

| | |
|---|---|
| Temp. (°C) | 825 |
| GHSV ($hr.^{-1}$) | 1200 |
| % $CH_4$ Conv. | 25.7 |
| % $C_2$ + Sel. | 0 |
| % $CO_x$ Sel. | 99.0 |
| % Coke Sel. | 1.0 |
| % Carbonate Sel. | —— |

Example 12

A contact solid comprising $NaMnO_2$/Ce oxide was prepared by impregnating $CeO_2$ with the appropriate amount of sodium permanganate from a water solution. The calcined solids contained the equivalent of 10 wt.% $NaMnO_2$. Results reported below in Table X are based on analyses of cumulative samples collected during two-minute methane runs at a temperature of 825°C and 600 $hr.^{-1}$ GHSV.

TABLE X

| | |
|---|---|
| % $CH_4$ Conv. | 23.4 |
| % $C_2$ + Sel. | 20.4 |
| % $CO_x$ Sel. | 45.3 |
| % Coke Sel. | 34.0 |
| % Carbonate Sel. | — |

Example 13

A silica-supported solid was prepared by sequential impregnation, drying and calcination of the following components on a silica support: Na (provided as sodium acetate), Mn (provided as manganese acetate), and Ce provided as $Ce(OH)_3$. Drying and calcination steps were preformed as described in Example 10. The finished, calcined solid contained 10 wt.% Ce/10wt. % Mn/1.7 wt% $Na/SiO_2$. When contacted with methane at 825°C and 600 $hr.^{-1}$ GHSV, the following results were obtained: 10.2% methane conversion and 86.1% $C_2$+ hydrocarbon selectivity.

Example 14

A contact solid was prepared by slurrying 25 grams (0.108 moles) of reagent grade $Fe_3O_4$ with 25 ml. of an aqueous solution containing 12.6 grams (0.093 moles) of reagent grade $Na(CH_3COO)\cdot 3H_2O$. The slurry was dried at room temperature for one hour and then at 120°C for two hours. It was then calcined at 850°C for 16 hours in static air. On a weight basis, the material was analyzed to be 7.6% sodium with an Fe:Na ratio of 3.5.

A quartz-tube reactor (described above) was packed with 5 ml. of 12—28 mesh particles of the solid. The results obtained over a series of methane contact runs are described in Table XI below.

TABLE XI

| Run# | Temp. (°C) | GHSV ($hr^{-1}$) | % Conv. | % Selectivity To: | | | |
|---|---|---|---|---|---|---|---|
| | | | | $C_2$+ | CO | $CO_2$ | Coke |
| 1 | 600 | 860 | 0.5 | 15.4 | 0 | 84.6 | 0 |
| 2 | 600 | 860 | 0.4 | 0 | 0 | 100 | 0 |
| 3 | 700 | 860 | 2.5 | 5.1 | 0 | 94.9 | 0 |
| 4 | 700 | 860 | 1.8 | 14.3 | 0 | 84.9 | 0.8 |
| 5 | 800 | 860 | 14.6 | 40.8 | 0 | 57.7 | 1.5 |
| 6 | 800 | 860 | 23.5 | 28.3 | 0 | 70.9 | 0.8 |
| 7 | 825 | 860 | 26.7 | 26.4 | 0 | 71.6 | 2.1 |
| 8 | 825 | 860 | 29.4 | 26.9 | 0 | 71.6 | 1.5 |
| 9 | 825 | 1200 | 21.2 | 33.9 | TR | 64.7 | 1.4 |
| 10 | 825 | 1200 | 25.5 | 29.0 | TR | 70.3 | 0.7 |
| 11 | 825 | 2400 | 10.7 | 42.9 | 0 | 55.6 | 1.6 |
| 12 | 825 | 2400 | 10.4 | 46.4 | 0 | 52.9 | 0.7 |
| 13 | 825 | 3600 | 6.4 | 47.4 | 0 | 51.4 | 1.2 |
| 14 | 825 | 3600 | 6.5 | 48.7 | 0 | 50.6 | 0.7 |
| 15 | 800 | 860 | 16.4 | 31.9 | 0 | 66.3 | 1.8 |
| 16 | 800 | 1200 | 11.0 | 38.2 | 0 | 60.2 | 1.6 |
| 17 | 800 | 1200 | 10.6 | 37.6 | 0 | 61.1 | 1.3 |

Example 15

A solid containing 7.3 wt.% Na and having an Fe:Na ratio of 3.5 was prepared as described in Example 14 except that reagent grade $Fe_2O_3$ was substituted for $Fe_3O_4$. Methane contact runs were performed as described above and results obtained are described in Table XII below. It is apparent from the data that the solid prepared from $Fe_2O_3$ does not perform as well as the solid prepared from $Fe_3O_4$.

TABLE XII

| Run# | Temp. (°C) | GHSV ($hr^{-1}$) | % Conv. | % Selectivity To: | | | |
|---|---|---|---|---|---|---|---|
| | | | | $C_2$+ | CO | $CO_2$ | Coke |
| 1 | 600 | 860 | 0.8 | 13.2 | 0 | 38.7 | 48.1 |
| 2 | 600 | 860 | 0.5 | 11.6 | 0 | 62.1 | 26.4 |
| 3 | 700 | 860 | 2.9 | 27.7 | 0 | 65.1 | 7.2 |
| 4 | 700 | 860 | 4.0 | 33.2 | 0 | 63.7 | 3.1 |
| 5 | 800 | 860 | 28.3 | 19.4 | 0 | 79.8 | 0.7 |
| 6 | 800 | 860 | 27.0 | 19.2 | 0 | 80.2 | 0.6 |
| 7 | 800 | 1200 | 20.3 | 25.2 | 0 | 73.2 | 1.6 |
| 8 | 800 | 1200 | 20.3 | 25.0 | 0 | 74.0 | 1.1 |
| 9 | 825 | 1200 | 26.4 | 21.0 | 0 | 78.2 | 0.8 |
| 10 | 825 | 1200 | 24.4 | 22.8 | O | 76.5 | 0.7 |
| 11 | 825 | 2400 | 13.5 | 28.4 | 0 | 69.2 | 1.9 |
| 12 | 825 | 2400 | 10.5 | 45.9 | 0 | 53.2 | 0.9 |
| 13 | 825 | 3600 | 7.1 | 38.5 | 0 | 60.1 | 1.4 |
| 14 | 825 | 3600 | 6.8 | 45.3 | 0 | 53.9 | 0.8 |
| 15 | 850 | 3600 | 9.5 | 29.9 | 0 | 69.9 | 0.2 |
| 16 | 850 | 3600 | 8.6 | 37.4 | 0 | 61.9 | 0.7 |

Example 16 (Comparative)

To demonstrate the promotional effect of materials such as sodium, methane contact runs were made using only $Fe_3O_4$ as the contact solid. Results are described in Table XIII below. In the absence of promoter material, methane conversion is greater but the selectivity to higher hydrocarbons is lower.

TABLE XIII

| Run# | Temp. (°C) | GHSV ($hr^{-1}$) | % Conv. | % Selectivity To: | | | |
|---|---|---|---|---|---|---|---|
| | | | | $C_2$+ | CO | $CO_2$ | Coke |
| 1 | 600 | 860 | 1.4 | 0 | 0 | 100 | 0 |
| 2 | 700 | 860 | 20.1 | .4 | 0 | 98.3 | 1.3 |
| 3 | 700 | 860 | 18.0 | .6 | 0 | 98.1 | 1.4 |
| 4 | 800 | 860 | 52.9 | .3 | 0 | 98.8 | 1.0 |
| 5 | 800 | 860 | 25.3 | 5.4 | 0 | 92.4 | 1.7 |
| 6 | 825 | 860 | 36.6 | 4.1 | 0 | 95.4 | 0.5 |
| 7 | 825 | 1200 | 22.8 | 6.5 | 0 | 92.3 | 1.2 |
| 8 | 825 | 2400 | 13.9 | 6.1 | 0 | 92.7 | 1.2 |
| 9 | 825 | 3600 | 9.4 | 6.0 | 0 | 93.4 | 0.7 |

Example 17

Three contact solids were prepared from $Fe_3O_4$, each with a different promoter (Li, Na or K). The iron to alkali metal ratio was 7 in each solid. Results obtained during methane contact runs over the Na-promoted solid are described in Table XIV. Results obtained during methane contact runs over the K-promoted solid are described in Table XV. Results obtained during methane contact runs over Li-promoted solids are described in Table XVI.

TABLE XIV

| Run# | Temp. (°C) | GHSV ($hr^{-1}$) | % Conv. | % Selectivity To: $C_2$+ | CO | $CO_2$ | Coke |
|---|---|---|---|---|---|---|---|
| 1 | 600 | 860 | 0.8 | 41.3 | 0 | 22.6 | 36.1 |
| 2 | 700 | 860 | 0.3 | 0 | 0 | 100 | TR |
| 3 | 700 | 860 | 3.4 | 17.0 | 0 | 79.0 | 3.9 |
| 4 | 700 | 860 | 3.8 | 22.6 | 0 | 74.8 | 2.5 |
| 5 | 800 | 860 | 30.7 | 12.4 | 0 | 87.0 | 0.6 |
| 6 | 800 | 860 | 27.0 | 17.3 | 0 | 81.9 | 0.8 |
| 7 | 800 | 1200 | 20.4 | 22.7 | 0 | 76.4 | 0.9 |
| 8 | 800 | 1200 | 18.9 | 23.5 | 0 | 74.6 | 1.9 |
| 9 | 800 | 2400 | 12.7 | 28.6 | 0 | 70.6 | 0.9 |
| 10 | 800 | 2400 | 12.2 | 28.7 | O | 70.6 | 0.7 |
| 11 | 825 | 2400 | 17.5 | 23.3 | 0 | 76.4 | 0.3 |
| 12 | 825 | 2400 | 13.9 | 28.3 | 0 | 71.0 | 0.7 |
| 13 | 835 | 3600 | 10.0 | 29.0 | 0 | 69.7 | 1.3 |
| 14 | 825 | 3600 | 9.5 | 28.6 | 0 | 70.9 | 0.5 |

TABLE XV

| Run# | Temp. (°C) | GHSV ($hr^{-1}$) | % Conv. | % Selectivity To: $C_2$+ | CO | $CO_2$ | Coke |
|---|---|---|---|---|---|---|---|
| 1 | 600 | 860 | 11.1 | 0.5 | 0 | 94.7 | 4.8 |
| 2 | 700 | 860 | 12.8 | 1.0 | 0 | 95.0 | 4.0 |
| 3 | 700 | 860 | 36.1 | 0.3 | 0 | 98.6 | 1.1 |
| 4 | 800 | 860 | 46.3 | 0.3 | 0 | 98.1 | 1.6 |
| 5 | 800 | 860 | 45.5 | 0.4 | 0 | 97.8 | 1.8 |
| 6 | 800 | 4800 | 7.7 | 2.6 | 0 | 96.6 | 0.8 |

TABLE XVI

| Run# | Temp. (°C) | GHSV ($hr^{-1}$) | % Conv. | % Selectivity To: $C_2$+ | CO | $CO_2$ | Coke |
|---|---|---|---|---|---|---|---|
| 1 | 600 | 860 | 2.1 | 0 | 0 | 100 | 0 |
| 2 | 700 | 860 | 17.6 | 0.5 | 0 | 48.5 | 0.4 |
| 3 | 700 | 860 | 27.3 | 0.2 | 0 | 99.1 | 0.6 |
| 4 | 800 | 860 | 66.1 | 0.2 | 0 | 99.4 | 0.5 |
| 5 | 800 | 860 | 54.7 | 0.5 | 0 | 98.5 | 0.9 |
| 6 | 700 | 860 | 15.7 | 0.7 | 0 | 47.5 | 1.8 |
| 7 | 700 | 1200 | 15.5 | 0.5 | 0 | 98.8 | 0.7 |
| 8 | 700 | 4800 | 5.1 | 0.7 | 0 | 98.1 | 1.2 |

**Claims**

1. A method for converting methane to higher hydrocarbon products which comprises contacting a gas comprising methane at elevated temperature with a contact solid comprising a reducible metal oxide characterised in that said reducible metal oxide comprises at least one reducible oxide of at least one metal selected from Pr, Tb and Ru and in that said contacting is effected at a temperature within the range of 700 to 900°C.

2. A method as claimed in claim 1 characterised in that said method includes the steps of:

(a) effecting said contacting in the substantial absence of catalytically effective Ni, Rh, Pd, Ag, Os, Ir, Pt, Au and compounds thereof and producing $C_{2+}$ hydrocarbons, coproduct water and solids comprising reduced metal oxide;

(b) recovering $C_{2+}$ hydrocarbons;

(c) at least periodically contacting solids comprising reduced metal oxide with an oxygen-containing gas to produce solids comprising reducible metal oxide; and

(d) contacting a gas comprising methane with solids produced in step (c) as recited in step (a).

3. A method as claimed in Claim 2, wherein the contacting of step (c) is carried out at a temperature within the range of 300 to 1200°C.

4. A method as claimed in Claim 2 or Claim 3, wherein the contacting of step (a) and the contacting of step (c) are carried out in different contact zones.

5. A method as claimed in any one of Claims 1 to 4, wherein said at least one reducible oxide is associated with a support material.

6. A method for converting methane to higher hydrocarbon products which comprises contacting a gas comprising methane at elevated temperature with a contact solid comprising a reducible metal oxide, characterised in that said contact solid comprises (i) at least one reducible oxide of at least one metal selected from Pr, Tb, Ce, Fe and Ru, and (ii) at least one promoter selected from alkali metals, alkaline earth metals, and compounds thereof, provided that where said at least one reducible oxide provided solely by iron oxide the atomic ratio of Fe to alkali metal and/or alkaline earth metal provided by said promoter is at least one and in that said contacting is effected at a temperature in the range of 500°C to 1000°C.

7. A method for synthesizing hydrocarbons from a methane source by contacting a gas containing methane at elevated temperature with a reducible metal oxide characterised in that said method comprises:

(a) contacting at a temperature within the range of 500 to 1000°C a gas comprising methane and a contact solid comprising (i) at least one reducible oxide (as herein defined) of at least one metal selected from Pr, Tb, Ce, Fe, and Ru; and (ii) at least one promoter selected from alkali metals, alkaline earth metals, and compounds thereof, said contacting producing $C_{2+}$ hydrocarbons, c-product water and solids comprising reduced metal oxide;

(b) recovering $C_{2+}$ hydrocarbons;

(c) at least periodically contacting solids comprising reduced metal oxide with an oxygen-containing gas to produce solids comprising reducible metal oxide; and

(d) contacting a gas comprising methane with solid produced in step (c) as recited in step (a).

8. A method as claimed in Claim 7, wherein the contacting of step (c) is carried out at a temperature within the range of 300 to 1200°C.

9. A method as claimed in Claim 7 or Claim 8, wherein the contacting of step (a) and the contacting of step (c) are carried out in different contact zones.

10. A method as claimed in any one of Claims 6 to 9, wherein where said at least one metal comprises Pr, the atomic ratio of Pr to alkali and/or alkaline earth metal in said solid is within the range of about 1—15:1; where said at least one metal comprises Tb, the atomic ratio of Tb to alkali and/or alkaline earth metal in said solid is within the range of 1—15:1; where said at least one metal comprises Ce, the atomic ratio of Ce to alkali and/or alkaline earth metal in said solid is within the range of 1—15:1; where said at least one metal comprises Fe, the atomic ratio of Fe to alkali and/or alkaline earth metal in said solid is within the range of 2—10:1; and where said at least one metal comprises Ru, the atomic ratio of Ru to alkali and/or alkaline earth metal in said solid is within the range of 2—10:1.

11. A method as claimed in any one of Claims 6 to 10, wherein when said at least one reducible metal oxide comprises an oxide of Pr, Ce or Tb said promoter is selected from Na, Li and compounds thereof, and when said at least one reducible metal oxide comprises an oxide of Fe or Ru, said promoter is selected from Na and compounds thereof.

12. A method as claimed in any one of Claims 6 to 11, wherein the contact solid is described by the empirical formula:

$$A_a\, B_b\, C_c\, P_d\, O_e$$

wherein A is at least one metal selected from Pr, Tb, Ce, Fe, Ru and mixtures thereof; B is a promoter selected from alkali metals, alkaline earth metals and mixtures thereof; C is selected from Mn, Sn, In, Ge, Pb, Sb, Bi and mixtures thereof; a, b, c, d and e indicate the atomic ratio of each component; and when a is 10, b is within the range of about 0.5—10, c is within the range of about 0—10, d is within the range of about

0—10, and e has a value which is determined by the valences and proportions of the other elements present.

13. A method as claimed in any one of Claims 6 to 12, wherein the components of the contact solid are associated with a support material.

14. A method as claimed in any one of Claims 6 to 12, wherin said at least one reducible oxide provides a support for the other components of said solid.

15. A method as claimed in any one of Claims 6 to 14, wherein the solid is contacted with a gas comprising methane at a temperature within the range of about 700 to 900°C.

16. A method as claimed in any one of Claims 1 to 15, wherein the gas comprising methane contains from 40 to 100 volume percent methane.

17. A method as claimed in any one of Claims 1 to 15, wherein the gas comprising methane contains from 80 to 100 volume percent methane.

18. A method as claimed in any one of Claims 1 to 15, wherein the gas comprising methane contains from 90 to 100 volume percent methane.

19. A method as claimed in any one of Claims 1 to 18, wherein the gas comprising methane is natural gas.

**Patentansprüche**

1. Verfahren zur Umwandlung von Methan in höhere Kohlenwasserstoffprodukte, bei dem man ein Gas, das Metahn enthält, bei erhöhter Temperatur mit einem Kontaktfeststoff, der ein reduzierbares Metalloxid enthält, in Kontakt bringt, dadurch gekennzeichnet, daß das reduzierbare Metalloxid mindestens ein reduzierbares Oxid mindestens eines Metalls, ausgewählt aus Pr, Tb und Ru, enthält und dadurch, daß das Inkontaktbringen bei einer Temperatur innerhalb des Bereiches von 700 bis 900°C durchgeführt wird.

2. Verfahren nach Anspruch 1, dadurch gekennzeichnet, daß das Verfahren die folgenden Schritte enthält:

(a) Durchführung des Inkontaktbringens in wesentlicher Abwesenheit von katalytisch wirksamen Ni, Rh, Pd, Ag, Os, Ir, Pt, Au und deren Verbindungen und Herstellung von $C_{2+}$-Kohlenwasserstoff, Wasser als Nebenprodukt und Feststoffen, die ein reduziertes Metalloxid enthalten;

(b) Zurückgewinnung von $C_{2+}$-Kohlenwasserstoffen;

(c) zumindest periodisches Inkontaktbringen von Feststoffen, die ein reduziertes Metalloxid enthalten, mit einem sauerstoffhaltigen Gas, um Feststoffe herzustellen, die ein reduzierbares Metalloxid enthalten, und

(d) Inkontaktbringen eines Gases, das Methan enthält, mit den in Stufe (c) hergestellten Feststoffen, wie in Stufe (a) beschrieben.

3. Verfahren nach Anspruch 2, bei dem das Inkontaktbringen der Stufe (c) bei einer Temperatur innerhalb des Bereiches von 300 bis 1200°C durchgeführt wird.

4. Verfahren nach Anspruch 2 oder 3, bei dem das Inkontaktbringen der Stufe (a) und das Inkontaktbringen der Stufe (c) in verschiedenen Kontaktzonen durchgeführt wird.

5. Verfahren nach einem der Ansprüche 1 bis 4, bei dem mindestens ein reduzierbares Oxid mit einem Trägermaterial verbunden ist.

6. Verfahren zur Unwandlung von Methan in höhere Kohlenwasserstoffprodukte, bei dem man ein Gas, das Methan enthält, bei erhöhter Temperatur mit einem Kontaktfeststoff, der ein reduzierbares Metalloxid enthält, in Kontakt bringt, dadurch gekennzeichnet, daß der Kontaktfeststoff (1) mindestens ein reduzierbares Oxid mindestens eines Metalls, ausgewählt aus Pr, Tb, Ce, Fe und Ru und (ii) mindestens einen Promotor, ausgewählt aus Alkalimetallen, Erdalkalimetallen und deren Verbindungen enthält, mit der Maßgabe, daß dann, wenn mindestens ein reduzierbares Metalloxid ausschließlich durch Eisenoxid zur Verfügung gestellt wird, das Atomverhältnis von Eisen zu Alkalimetall und/oder Erdalkalimetall, das durch den promotor zur Verfügung gestellt wird, mindestens eines ist und dadurch, daß das Inkontaktbringen bei einer Temperatur im Bereich von 500°C bis 1000°C durchgeführt wird.

7. Verfahren zur Synthese von Kohlenwasserstoffen aus einem Methanausgangsmaterial durch Inkontaktbringen eines Gases, das Methan enthält, bei erhöhter Temperatur mit einem reduzierbaren Metalloxid, dadurch gekennzeichnet, daß man

(a) ein Gas, das Methan enthält, bei einer Temperatur innerhalb des Bereiches von 500°C bis 1000°C mit einem Kontaktfeststoff in Kontakt bringt, der (i) mindestens ein reduzierbares Oxid (wie vorstehend definiert) mindestens eines Metalls, ausgewählt aus Pr, Tb, Ce, Fe und Ru und (ii) mindestens einen Promotor, ausgewählt aus Alkalimetallen, Erdalkalimetallen und deren Verbindungen, enthält, wobei durch das Inkontaktbringen $C_{2+}$-Kohlenwasserstoffe, Wasser als Nebenprodukt und Feststoffe, die ein reduziertes Metalloxid enthalten werden;

(b) $C_{2+}$-Kohlenwasserstoffe zurückgewinnt;

(c) mindestens periodisch Kontaktfeststoffe, die ein reduziertes Metalloxid enthalten, mit einem sauerstoffhaltigen Gas, in Kontact bringt, um Feststoffe, die ein reduzierbares Metalloxid enthalten, herzustellen und

(d) ein Methan enthaltendes Gas mit den in Stufe (c) hergestellten Festoffen, wie in Stufe (a) beschrieben, in Kontakt bringt.

8. Verfahren nach Anspruch 7, bei dem das Inkontaktbringen der Stufe (c) bei einer Temperatur innerhalb des Bereiches von 300°C bis 1200°C durchgeführt wird.

9. Verfahren nach Anspruch 7 oder 8, bei dem das Inkontaktbringen der Stufe (a) und das Inkontaktbringen der Stufe (c) in verschiedenen Kontaktzonen durchgeführt wird.

10. Verfahren nach einem der Ansprüche 6 bis 9, bei dem, wenn mindestens ein Metall Pr enthält, das Atomverhältnis von Pr zu Alkali- und/oder Erdalkalimetallen in dem Feststoff innerhalb des Bereiches von etwa 1—15:1 beträgt; wenn mindestens ein Metall Tb enthält, das Atomverhältnis von Tb zu Alkali- und/oder Erdalkalimetallen im Feststoff innerhalb des Bereiches von 1—15:1 liegt; wenn mindestens ein Metall Ce enthält, das Atomverhältnis von Ce zu Alkali- und/oder Erdalkimetallen im Feststoff innerhalb des Bereiches von 1—15:1 liegt; wenn mindestens ein Metall Fe enthält, das Atomverhältnis von Fe zu Alkali- und/oder Erdalkalimetallen im Feststoff innerhalb des Bereiches von 2—10:1 liegt; und wenn mindestens ein Metall Ru enthält, das Atomverhältnis von Ru zu Alkali- und/oder Erdalkalimetallen im Feststoff innerhalb des Bereiches von 2—10:1 liegt.

11. Verfahren nach einem der Ansprüche 6 bis 10, bei dem mindestens ein reduzierbares Metalloxid ein Oxid von Pr, Ce oder Tb enthält, der Promotor aus Na, Li und deren Verbindungen gewählt ist, und wenn das mindestens eine reduzierbare Metalloxid ein Oxid von Fe oder Ru enthält, der Promotor aus Na und dessen Verbindungen gewählt wird.

12. Verfahren nach einem der Ansprüche 6 bis 11, bei dem der Kontaktfeststoff durch die folgende empirische Formel beschrieben wird:

$$A_aB_bC_cP_dO_e$$

in der A mindestens ein Metall, ausgewählt aus Pr, Tb, Ce, Fe, Ru und deren Mischungen ist; B ein Promotor, ausgewählt aus Alkalimetallen, Erdalkalimetallen und deren Mischungen ist; C aus Mn, Sn, In, Ge, Pb, Sb, Bi und deren Mischungen ausgewählt ist; a, b, c, d und e das Atomverhältnis jeder Komponente angeben; und wenn a 10 ist, b innerhalb des Bereiches von etwa 0,5—10, c innerhalb des Bereiches von etwa 0—10, d innerhalb des Bereiches von etwa 0—10 liegt, und e einen Wert besitzt, der durch die Valenzen und Anteile der anderen vorhandenen Elemente bestimmt wird.

13. Verfahren nach einem der Ansprüche 6 bis 12, bei dem die Komponenten des Kontaktfeststoffes mit einem Trägermaterial verbunden sind.

14. Verfahren nach einem der Ansprüche 6 bis 12, bei dem mindestens ein reduzierbares Oxid einen Träger für die anderen Komponenten des Feststoffes zur Verfügung stellt.

15. Verfahren nach einem der Ansprüche 6 bis 14, bei dem der Feststoff mit dem Methan enthaltenden Gas bei einer Temperatur innerhalb des Bereiches von etwa 700°C bis 900°C in Kontakt gebracht wird.

16. Verfahren nach einem der Ansprüche 1 bis 15, bei dem das Methan enthaltende Gas 40 bis 100 vol.-% Methan enthält.

17. Verfahren nach einem der Ansprüche 1 bis 15, bei dem das Methan enthaltende Gas 80 bis 100 Vol.-% Methan enthält.

18. Verfahren nach einem der Ansprüche 1 bis 15, bei dem das Methan enthaltende Gas 90 bis 100 Vol.-% Methan enthält.

19. Verfahren nach einem der Ansprüche 1 bis 18, bei dem das Methan enthaltende Gas Erdgas ist.

**Revendications**

1. Procédé pour convertir du méthane en produits hydrocarbonés supérieurs, comprenant la mise en contact d'un gaz contenant du méthane à une température élevée avec une masse de contact solide comprenant un oxyde métallique réductible, caractérisé en ce que cet oxyde métallique réductible comprend au moins un oxyde réductible d'au moins un métal choisi parmi Pr, Tb et Ru et que ce contact est effectué à une température dans la gamme de 700 à 900°C.

2. Procédé suivant la revendication 1, charactérisé en ce qu'il comprend les étapes suivantes:

(a) réalisation de cette mise en contact en l'absence pratique de Ni, Rh, Pd, Ag, Os, Ir, Pt, Au et de leurs composés efficaces catalytiquement et production d'hydrocarbures en $C_{2+}$, d'eau coproduite et de solides comprenant l'oxyde métallique réductible;

(b) récupération des hydrocarbures en $C_{2+}$;

(c) mise en contact au moins périodique de solides comprenant un oxyde métallique réduit avec un gaz contenant de l'oxygène pour produire des solides comprenant un oxyde métallique réductible; et

(d) mise en contact d'un gaz contenant du méthane avec des solides produits dans l'étape (c) de la façon indiquée dans l'étape (a).

3. Procédé suivant la revendication 2, charactérisé en ce que la mise en contact de l'étape (c) est effectuée à une température dans la gamme de 300 à 1200°C.

4. Procédé suivant la revendication 2 ou la revendication 3, caractérisé en ce que la mise en contact de l'étape (a) et la mise en contact de l'étape (c) sont effectuées dans des zones de contact différentes.

5. Procédé suivant l'une quelconque des revendications 1 à 4, caractérisé en ce que cet oxyde réductible au moins présent est associé à un matériau de support.

6. Procédé pour convertir du méthane en produits hydrocarbonés supérieurs comprenant la mise en contact d'un gaz comprenant du méthane à une température élevée avec une mass de contact solide comprenant un oxyde métallique réductible, caractérisé en ce que cette masse de contact solide comprend (i) au moins un oxyde réductible d'au moins un métal choisi parmi Pr, Tb, Ce, Fe et Ru et (ii) au moins un activateur choisi parmi des métaux alcalins, des métaux alcalino-terreux et leurs composés, à condition que dans le cas où cet oxyde réductible au moins présent est fourni uniquement par l'oxyde de fer, le rapport atomique du Fe au métal alcalin et/ou au métal alcalino-terreux fournis par l'activateur soit au moins égal à 1, et que ce contact est effectué à une température dans la gamme de 500 à 1000°C.

7. Procédé pour synthétiser des hydrocarbures à partir d'une source de méthane par contact d'un gaz contenant du méthane à une température élevée avec un oxyde métallique réductible, caractérisé en ce qu'il comprend:

(a) la mise en contact à une température dans la gamme de 500°C à 1000°C d'un gaz comprenant du méthane avec une masse de contact solide comprenant (i) au moins un oxyde réductible (tel que défini ici) d'au moins un métal choisi parmi Pr, Tb, Ce, Fe et Ru; et (ii) au moins un activateur choisi parmi des métaux alcalins, des métaux alcalinoterreux et leurs composés, ce contact produisant des hydrocarbures en $C_{2+}$, de l'eau coproduite et des solides comprenant l'oxyde métallique réduit;

(b) la récupération des hydrocarbures en $C_{2+}$;

(c) la mise en contact au moins périodiquement des solides comprenant un oxyde métallique réduit avec un gaz contenant de l'oxygène pour produire des solides comprenant un oxyde métallique réductible; et

(d) mise en contact d'un gaz comprenant du méthane avec les solides produits dans l'étape (c) suivant la procédure de l'étape (a).

8. Procédé suivant la revendication 7, caractérisé en ce que la mise en contact de l'étape (c) est effectuée à une température dans la gamme de 300 à 1200°C.

9. Procédé suivant la revendication 7 ou la revendication 8, caractérisé en ce que la mise en contact de l'étape (a) et la mise en contact de l'étape (c) sont effectuées dans des zones de contact différentes.

10. Procédé suivant l'une quelconque des revendications 6 à 9, caractérisé en ce que dans le cas où ce métal au moins présent est le Pr, le rapport atomique du Pr au métal alcalin et/ou alcalino-terreux dans cette masse solide est dans la gamme d'environ 1—15:1; dans le cas où ce métal au moins présent est le Tb, le rapport atomique du Tb au métal alcalin et/ou alcalino-terreux dans ce solide est dans la gamme de 1—15:1; dans le cas où ce metal au moins présent est le Ce, le rapport atomique du Ce au métal alcalin et/ou alcalino-terreux dans ce solide est dans la gamme de 1—15:1; dans le cas où ce métal au moins présent est le Fe, la rapport atomique du Fe au métal alcalin et/ou alcalino-terreux dans ce solide est dans la gamme de 2—10:1; et dans le cas où ce métal au moins présent est le Ru, le rapport atomique du Ru au métal alcalin et/ou alcalino-terreux dans ce solide est dans le gamme de 2—10:1.

11. Procédé suivant l'une quelconque des revendications 6 à 10, caractérisé en ce que lorsque cet oxyde métallique réductible au moins présent comprend un oxyde de Pr, Ce ou Tb, cet activateur est choisi parmi Na, Li et leurs composés et lorsque cet oxyde métallique réductible au moins présent comprend un oxyde de Fe ou Ru, cet activateur est choisi parmi Na et ses composés.

12. Procédé suivant l'une quelconque des revendications 6 à 11, caractérisé en ce que la masse solide de contact est représentée par la formule empirique:

$$A_aB_bC_cP_dO_e$$

dans laquelle A est au moins un métal choisi parmi Pr, Tb, Ce, Fe, Ru et leurs mélanges; B est un activateur choisi parmi des métaux alcalins, des métaux alcalino-terreux et leurs mélanges, C est choisi parmi Sn, In, Ge, Pb, Sb, Bi et leurs mélanges; a, b, c, d et e indiquent le rapport atomique de chaque composant; et lorsque a est 10, b est dans la gamme d'environ 0,5—10, c est dans la gamme d'environ 0—10, d est dans la gamme d'environ 0—10, et e a une valeur qui est déterminée par les valences et les proportions des autres éléments présents.

13. Procédé suivant l'une quelconque des revendications 6 à 12, caractérisé en ce que les composants de la masse de contact solide sont associés à un matériau de support.

14. Procédé suivant l'une quelconque des revendications 6 à 12, caractérisé en ce que cet oxyde réductible au moins présent fournit un support pour les autres composants de ce solide.

15. Procédé suivant l'une quelconque des revendications 6 à 14, caractérisé en ce que le solide est mis en contact avec un gaz comprenant du méthane à une température dans la gamme d'environ 700 à 900°C.

16. Procédé suivant l'une quelconque des revendications 1 à 15, caractérisé en ce que le gaz comprenant du méthane contient 40 à 100% en volume de méthane.

17. Procédé suivant l'une quelconque des revendications 1 à 15, caractérisé en ce que le gaz comprenant du méthane contient 80 à 100% en volume de méthane.

18. Procédé suivant l'une quelconque des revendications 1 à 15, caractérisé en ce que le gaz comprenant du méthane contient de 90 à 100% en volume de méthane.

19. Procédé suivant l'une quelconque des revendications 1 à 18, caractérisé en ce que le gaz comprenant du méthane est du gaz naturel.